# EUROPÄISCHE PATENTANMELDUNG

(11) **EP 2 018 887 A1**
(43) Veröffentlichungstag der Anmeldung: **28.01.2009**
(21) Anmeldenummer: 08015905.6
(22) Anmeldetag: 10.01.2006
(51) Int. Cl.: A61N 5/10

(54) **Implantat zur Behandlung der Innenwände eines Resektionshohlraumes**

(62) Teilanmeldung aus: 06000430.6
(71) Anmelder: Acrostak Corp. BVI, 8409 Winterthur (CH)
(72) Erfinder: Popowski, Youri, 1206 Genf (CH); Berger, Erwin, 9506 Stettfurt (CH)
(74) Vertreter: Weiss, Wolfgang

(57) **Zusammenfassung**

Die Erfindung betrifft ein Implantat 1 mit röntgenologisch erkennbaren Indikatoren 26 zur Behandlung von einem Hohlraum infolge einer Resektion, wobei das Implantat 1 modular aufgebaut ist und die Module miteinander verbindbar sind.

## Beschreibung

Die Erfindung betrifft ein Implantat gemäss des Oberbegriffs von Anspruch 1.

Die Behandlung eines Gewebes innerhalb eines Hohlraumes infolge eines chirurgischen Eingriffs zur Entfernung eines Tumors hat in den vergangenen Jahren enorm an Bedeutung gewonnen. Der Gegenstand dieser Erfindung ist eine Einrichtung zur Strahlungstherapie.

In der EP 1402922 A1 ist ein derartiges Implantat offenbart. Dieses Implantat betrifft eine aufblasbare Kammer mit Vorrichtungen zur Einführung einer Strahlenquelle

Die US 4,815,449 offenbart eine Vorrichtung dieser Gattung, wobei das Implantat aus biologisch abbaubarem Material besteht.

Die Druckschrift US 6,673,006 offenbart eine Vorrichtung zur Anwendung einer Strahlentherapie, insbesondere einer Strahlentherapie möglichst nahe an dem zu bestrahlenden Medium (Brachytherapie).

Weiterer Stand der Technik ist aus den Druckschriften US 6,413,204, US 6,083,148, US 6,022,308, US 4,763,642 und US 5,913,813 bekannt.

Die Aufgabe der Erfindung besteht darin, ein Implantat vorzuschlagen, welches röntgenologisch erkennbar ist und auf eine Vielzahl von Anwendungsgebieten der Strahlentherapie einsetzbar ist.

Eine weitere Aufgabe der Erfindung ist es, einige Bereiche, insbesondere die äusseren Dimensionen röntgenologisch zu erkennen.

Die Erfindung schlägt eine neue Vorrichtung zur Positionierung einer Strahlungsquelle zur Behandlung der inneren Wände eines Resektionshohlraumes vor. Zum Beispiel ist diese Vorrichtung anwendbar auf Resektionshohlräume in der Brust, Prostata, Gehirn oder andern zu behandelnden Resektionshohlräumen im menschlichen Körper, die infolge einer Entfernung eines Tumors entstanden sind.

Erfindungsgemäss wird die Aufgabe durch das Kennzeichen von Anspruch 1 gelöst.

Bei manchen Anwendungen ist es erforderlich, dass das Implantat röntgenologisch erkennbar ist, beispielsweise im Rahmen einer üblichen CT Behandlung. Es ist daher vorgesehen, dem Material des Implantates, röntgenologisch erkennbare Indikatoren hinzuzufügen. Beispielsweise bestehen die röntgenologisch erkennbaren und biologisch abbaubaren Indikatoren aus Mg-, Ba-, Y-, Zr-, Sr-, Sc-, Ti-, Nb-, Fe-, Ag-, Yb,- Nd-, Gd- oder Ca-Legierungen und/oder Verbindungen.

Ebenso ist es gemäss der Erfindung möglich, den Führungskatheter für Röntgenstrahlung sichtbar zu machen, beispielsweise durch Impregnierung mit Bariumsulfat, mit Metalldrähten innerhalb der Katheterwände, einen Versteifungsdraht oder einen vollkommen aus Metall bestehenden Führungskatheter. Der Versteifungsdraht kann aus biologischabbaubarem Material, Kunststoff oder mit Kunststoff ummantelten Metalldraht bestehen.

Eine besonders bevorzugte Ausführungsform besteht darin, das für Röntgenstrahlung sichtbare Material aus einem biologisch abbaubaren Stoff zu bilden, beispielsweise aus Magnesium, Magnesiumlegierungen oder Magnesiumverbindungen.

Vorzugsweise sind die Indikatoren so an der Peripherie des Implantates angeordnet, dass die äusseren Umrisse des Implantates auf dem Röntgenmonitor (CT Scanner) zu erkennen sind, was erhebliche Vorteile für die Dosimetrie bringt. Die üblichen Durchmesser der Implantate liegen zwischen 1 cm und 5 cm, vorzugsweise 1.5 cm, 2.5 cm und 3.5 cm.

Gemäss einer besonders bevorzugten Ausführungsform besteht ein Teil oder das ganze Implantat aus einem biologisch abbaubaren Material, welches mit einem netzförmigen Gitter versehen ist. Das Gitter besteht aus Magnesium, einer Magnesiumlegierung oder einer Magnesiumverbindung zur Erkennung von Röntgenstrahlung (CT Scanner). Im Übrigen hat diese Ausführungsform den Vorteil, dass sie infolge von geeignet ausgewähltem elastischen Material für die Anwendung im menschlichen Körper geeignet ist.

Vorzugsweise sind das Implantat, relevante Teile des Implantates und/oder der Führungskatheter mit antibiotischem oder antiseptischem Material, beispielsweise Silber beschichtet.

Ausführungsbeispiele sind in der Zeichnung dargestellt. Es zeigen:
- Fig. 1 a und 1b: zwei zueinander passende Modulteile
- Fig.2: ein Modulpaar
- Fig.3: ein kettenförmiges Implantat
- Fig.10a: eine kettenförmiges Implantat mit Modulen nach Figur 8
- Fig. 11a und b: eine schematische Anordnung eines Implantates

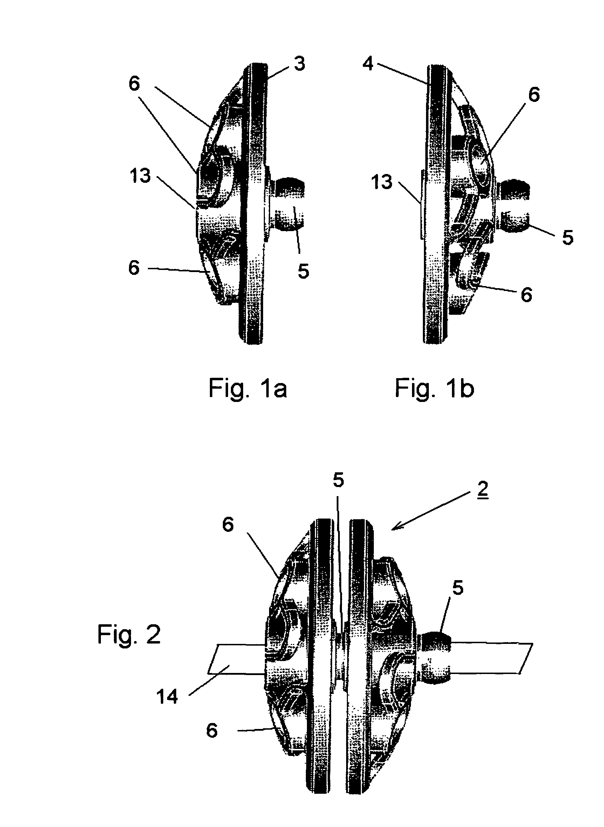

In den Figuren 1a und 1b sind zwei nach der Erfindung mögliche Modulteile 3 und 4 dargestellt. Die beiden Modulteile 3 und 4 sind im Umfang kalottenförmig ausgebildet, wobei im Zentrum der Modulteile 3 und 4 jeweils Steckverbindungen 5 angeordnet sind. Die Steckverbindungen können einstückig an den Modulteilen 3 und 4 angebracht sein. Ebenso sind separate Clips geeignet, die Modulteile steckbar zu einem Modulpaar 2 zusammenzufügen, wie es in Figur 2 gezeigt ist. Das Modulpaar 2 bildet die kleinstmögliche Ausführungsform eines erfindungsgemässen Implantates 1. In den Modulteilen 3 und 4 sind Durchführungen 6 für Katheter oder Führungselemente für radiologische Quellen zur Behandlung der kranken Bereiche im Resektionshohlraum angeordnet. Der beispielsweise durch das Zentrum 13 geführte Katheter 14 ist nach der Behandlung heraus ziehbar, wobei er sich von den Modulpaaren (2, 9, 10, 11) löst.

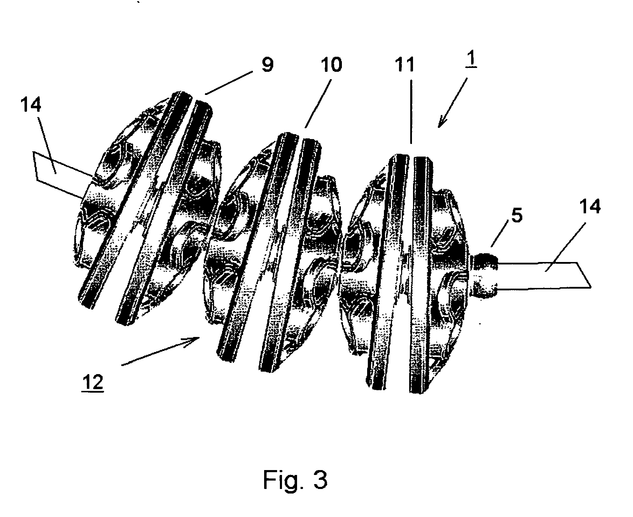

Die Figur 3 zeigt ein Implantat 1, welches aus drei Modulpaaren (9, 10, 11) aufgebaut ist und eine Kette 12 bildet. Die Modulpaare (9, 10, 11) bzw. die Kette 12 sind aus biologisch abbaubarem Material und bleiben nach der Behandlung in dem Hohlraum. Um eine radiologische Erkennung zu ermöglichen sind dem Material Stoffe beigefügt, die im Röntgenbild sichtbar werden. In Figur 4 ist das Implantat von innen zu sehen.

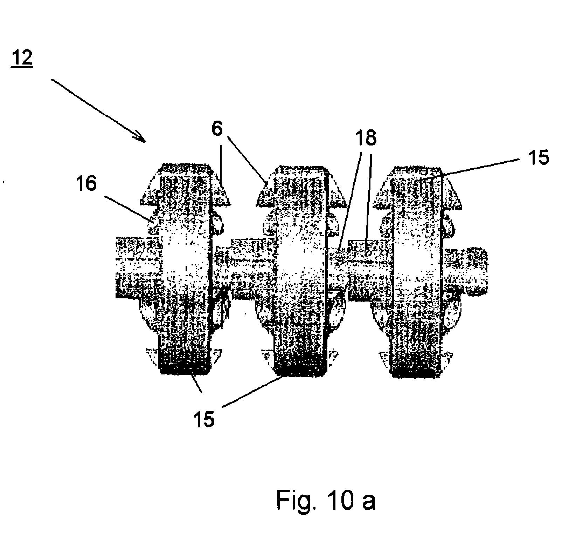

Die Figur 10a zeigt ein weiteres Ausführungsbeispiel eines Implantates 1. Dieses Implantat 1 besteht aus mindestens einem Modul 15, welches einstückig ausgebildet ist und wie die Modulpaare in dem vorherigen Ausführungsbeispiel Durchführungen 6 für die Katheter bzw. radiologischen Quellen zur Therapie aufweist. Im Inneren des Moduls 15 sind am Umfang des Aussenrings des Moduls 15 kleine Durchführungen 16 ausgebildet, die zur Einführung eines Stoffes, der radiologisch erkennbar ist, dient. Beispielsweise kann der Stoff aus Magnesiumlegierungen bestehen.

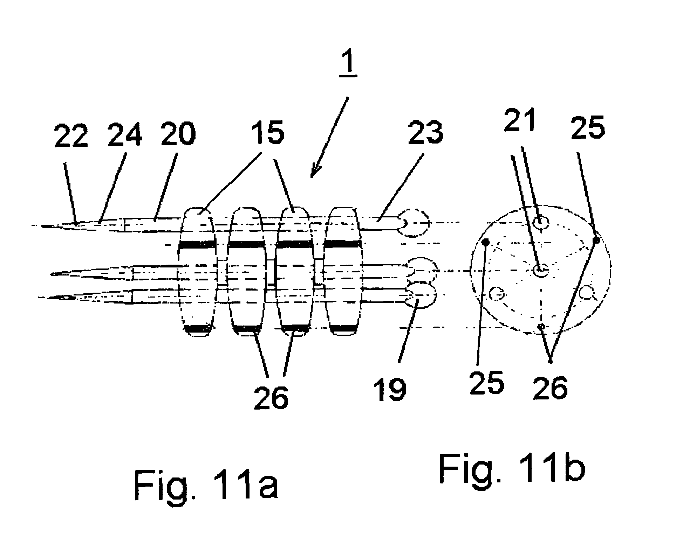

Ein erfindungsgemässes Implantatsystem 1 ist in den Figuren 11a (Seitenansicht) und 11b (Querschnitt) gezeigt. In Figur 11a sind vier Module 15 mit drei Führungskathetern 20 zu sehen, welche durch die Durchführungen 21 geführt sind. Die Führungskatheter 20 weisen am distalen Ende 22 Nadeln 24 und am proximalen Ende 23 Stopper 19 auf. Die Führungskatheter 20 bestehen aus biologisch abbaubarem und antiseptischem Material. In den Öffnungen 25 können beispielsweise Magnesiumstifte 26 zur Sichtbarmachung des Implantates auf einem CT-Monitor angeordnet sein.

### Bezugszeichenliste

- 1: Implantat
- 2: Modulpaar
- 3: Modulteil
- 4: Modulteil
- 5: Steckverbindung
- 6: Durchführung
- 9: Modulpaar
- 10: Modulpaar
- 11: Modulpaar
- 12: Kette
- 13: Zentrum
- 14: Katheter
- 15: Modul
- 16: Durchführungen
- 18: Steckverbindung
- 19: Stopper
- 20: Führungskatheter
- 21: Durchführung
- 22: Distales Ende
- 23: Proximales Ende
- 24: Nadel
- 25: Öffnung
- 26: Magnesiumstifte

## Patentansprüche

1. Implantat (1) zur Behandlung von einem Hohlraum infolge einer Resektion, wobei das Implantat in Form Modulpaaren (2, 9, 10, 11) oder Modulen (15) aufgebaut ist, wobei die **dadurch** gebildete Kette (12) eine solche elastische Flexibilität aufweist, dass sie den Bewegungen des Organs, in das das kettenförmige Implantat (12) eingesetzt ist, angepasst ist und dass es einen mit einem Stopper (19) versehenen Führungskatheter (20) aufweist, wobei der Führungskatheter (20) durch eine in den Modulpaaren (2, 9, 10, 11) oder Modulen (15) angeordnete Durchführung ein- und ausführbar ist, **dadurch gekennzeichnet, dass** die Modulteile (3, 4), die Modulpaare (2, 9, 10) oder die Module (15) Durchführungen (16) zur Zuführung oder Anordnung von röntgenologisch oder im CT-Verfahren erkennbaren und biologisch abbaubaren Indikatoren (26) aufweisen.

2. Implantat nach Anspruch 1, **dadurch gekennzeichnet, dass** die Indikatoren (26) für die Erkennung von Röntgenstrahlen so an der Peripherie des Implantates (1) angeordnet sind, dass der Aussenradius des Implantats auf dem Monitor messbar ist.

3. Implantat nach einem der Ansprüche 1 oder 2, **dadurch gekennzeichnet, dass** die Indikatoren (26) für die Erkennung von Röntgenstrahlen oder im CT-Verfahren so an der Peripherie des Implantates (1) angeordnet sind, dass die Geometrie des Implantates (1) erkennbar wird.

4. Implantat nach mindestens einem der Ansprüche 1, 2 oder 3, **dadurch gekennzeichnet, dass** das Material des Implantats (1) mittels eines netzförmigen Gitters armiert ist.

5. Implantat nach mindestens einem der vorherigen Ansprüche, **dadurch gekennzeichnet, dass** das netzförmige Gitter aus biologisch abbaubaren Magnesium, einer Magnesiumlegierung oder einer Magnesiumverbindung besteht.

6. Implantat nach mindesten einem der vorherigen Ansprüche, **dadurch gekennzeichnet, dass** die im Implantat verwendeten biologisch abbaubaren Materialien sich in etwa genau so schnell abbauen, wie die im Resektionshohlraum verwendeten chirurgischen Fäden.

7. Implantat, **dadurch gekennzeichnet, dass** es aus biologisch abbaubarem Material besteht und dass es mit Magnesiumstiften zur Erkennung im CT-Verfahren versehen ist.

8. Implantat nach Anspruch 7, **dadurch gekennzeichnet, dass** die Magnesiumstifte einen Durchmesser von 0,5 mm bis 5 mm, vorzugsweise 1,5 mm und eine Länge von 3mm bis 10mm, vorzugsweise 4mm aufweisen.

9. Implantat nach Anspruch 7 **dadurch gekennzeichnet, dass** es ein Magnesiumgitter aufweist.

10. Implantat nach den Ansprüchen 7, 8 oder 9, **dadurch gekennzeichnet, dass** das Magnesium biologisch abbaubar ist.
